# EUROPEAN PATENT SPECIFICATION

(11) **EP 3 834 861 B1**
(45) Date of publication and mention of the grant of the patent: **06.03.2024**
(21) Application number: 19216147.9
(22) Date of filing: 13.12.2019
(51) Int. Cl.: A61M 1/36, A61M 39/10, A61M 5/14, A61M 5/142, A61M 39/00

(54) **ADD-ON MODULE FOR AN APPARATUS FOR EXTRACORPOREAL TREATMENT OF BLOOD AND BLOOD SET PROVIDED WITH SAID ADD-ON MODULE**
ZUSATZMODUL FÜR EINE VORRICHTUNG ZUR EXTRAKORPORALEN BEHANDLUNG VON BLUT UND MIT BESAGTEM ZUSATZMODUL AUSGESTATTETER BLUTSATZ
MODULE COMPLÉMENTAIRE DE TRAITEMENT EXTRACORPOREL DU SANG ET CIRCUIT POUR LE SANG FOURNI AVEC LEDIT MODULE COMPLÉMENTAIRE

(43) Date of publication of application: 16.06.2021
(73) Proprietor: Gambro Lundia AB, 226 43 Lund (SE)
(72) Inventor: VERNIN, Guillaume, F-69330 MEYZIEU (FR)
(74) Representative: PGA S.p.A., Milano, Succursale di Lugano

(56) References cited:
- WO-A2-03/006944
- FR-A1- 2 889 451
- US-A1- 2011 230 772
- US-A1- 2013 110 028

## Description

### TECHNICAL FIELD

The invention relates to an add-on module for an apparatus for extracorporeal treatment of blood and to a blood set for an apparatus for extracorporeal treatment of blood provided with this add-on module.

Extracorporeal blood treatment involves removing blood from a patient, treating the blood externally to the patient, and returning the treated blood to the patient. Extracorporeal blood treatment is typically used to extract undesirable matter or molecules from the patient's blood and add desirable matter or molecules to the blood. Extracorporeal blood treatment is used with patients unable to effectively remove matter from their blood, such as when a patient has suffered temporary or permanent kidney failure. These patients and other patients may undergo extracorporeal blood treatment to add or remove matter to their blood, to maintain an acid/base balance or to remove excess body fluids, or to perform extracorporeal gas exchange processes, for example.

For instance, in a haemodialysis treatment a patient's blood and a treatment liquid approximately isotonic with blood flow are circulated in a respective compartment of haemodialyser, so that, impurities and undesired substances present in the blood (urea, creatinine, etc.) may migrate by diffusive transfer from the blood into the treatment liquid. The ion concentration of the treatment liquid is chosen so as to correct the ion concentration of the patient's blood. In a treatment by haemodiafiltration, a convective transfer by ultrafiltration, resulting from a positive pressure difference created between the blood side and the treatment-liquid side of the membrane of a haemodiafilter, is added to the diffusive transfer obtained by dialysis. Hemoperfusion, oxygenation or decarboxylation treatments are also known.

A vascular access is used to remove the patient's blood so that it is filtered through the blood treatment unit (dialyzer, filter) and then returned to the patient. In case of chronic treatments, the vascular access may be arterio-venous. Two needles may be inserted into the vein, one to draw blood and one to return it. The orientation of the needles takes the normal flow of the blood into account. The "arterial" needle draws blood from the "upstream" location while the "venous" needle returns blood "downstream". In case of acute treatments, the vascular access may be veno-venous, i.e. using a double-lumen catheter inserted in a central vein. The blood treatment unit comprises pressure monitoring devices, as to check, for example, for circuit obstructions.

### BACKGROUND OF THE INVENTION

In the field of extracorporeal blood treatments and therapies, some problems reported by users are issues at the vascular access. Access Extremely Negative (AEN) is one of the most encountered alarm in the field, mainly linked to catheter tip stuck against blood vessel's wall or, more rarely, to clots obstructing the access way of the catheter. When this alarm relates to catheter positioning inside the vein, the nurse has to get the access pressure back closer to an atmospheric level, e.g. through the use of a Y-connector connected to the access line, unstick the catheter tip from the vein wall and then try to move the catheter inside the vein and resume the treatment.

Another drawback is that at the end of the treatment, when blood is to be returned to the patient, one operator has to disconnect the patient access and connect a saline bag to the set's access line, in aseptic conditions, and another operator has to operate on the monitor of the machine in non-aseptic conditions. Therefore, two operators are needed for one patient.

Another drawback is that, as per the blood return described above, both saline and blood recirculation procedures currently require sterile and non-sterile interventions and the manual connection of supplementary devices (Y-connector, saline bag).

In all the cases detailed above, the operator has to handle the vascular access, to disconnect and eventually reconnect the same to the patient. All this implies operator workload and increased probability of occurrence for non-aseptic handling.

Furthermore, the use of a Y-connector connected to the access line involves further risks. On the access side, the user may forget to clamp the Y saline line prior to resuming the treatment. In case of blood pump stop and slightly negative patient access pressure, the saline bag content would be sucked into the catheter, causing air ingress leading to set clotting (or potentially air embolism). Additional modules, configured to be placed in-line between the blood treatment apparatus and the patient vascular access and configured to divert the flow of blood without necessarily disconnecting the patient, are also known.

For instance, document US 3, 626, 938 discloses a shunt valve device which is designed to be mounted permanently on a body member of a person and permanently connected to the artery and vein of the person. The device is provided with valve means for selectively connecting the artery and vein to an artificial kidney or the like. The device further has rotary shunt means for directing the flow of blood back to the body member when the device is not operatively connected to the artificial kidney.

Document US 5,894,011 discloses a device for selectively controlling the direction of blood flow to and from the patient during hemodialysis. The device comprises two interlocking disks that rotate in relation to each other without separating. The two disks have fluid fittings that allow the blood lines attached to the patient to connect to one of the disks and the blood inlet and outlet for the hemodialysis machine to connect to the other. The center of each fluid fitting is a channel that aligns to a corresponding channel in the other disk. The disks rotate between two fixed relative positions, referred to herein as preferred alignments. The preferred alignments are such that the line drawing blood from the patient in the first preferred alignment becomes the line returning blood to the patient in the second preferred alignment, and the line returning blood to the patient in the first preferred alignment becomes the line drawing blood from the patient in the second preferred alignment. A bypass channel allows blood to flow from the outlet to the inlet of the hemodialysis machine when the device is in neither of its two preferred alignments. <INSERT PAGE 4A>

These devices allow a limited number of flow path configurations and, due to their structure and geometry of the ducts, may cause pressure drops and/or clotting.

It is therefore an object of the present invention to provide an add-on module for an apparatus for extracorporeal treatment of blood, wherein the add-on module is configured to divert the flow of blood or other fluid/s in order to perform a plurality of procedures (e.g. all the steps of the treatment, pre and post treatment and possible troubleshooting procedures) without necessarily disconnecting the patient.

It is an object of the present invention to provide an add-on module which may be easily interposed between the apparatus for extracorporeal treatment of blood and the patient, featuring various functional components and able to be set in a plurality of configurations to perform the plurality of procedures.

In particular, it is an object providing an add-on module able to provide easy and safe AEN troubleshooting, blood return at the treatment end, blood recirculation, saline recirculation. DocumentUS20110230772A1 discloses a device and method for invasive blood pressure measurement in a vascular access under continuous blood flows in a treatment device in extracorporeal detoxification methods. Systemic arterial pressure is directly measured using an existing vascular access for dialysis or in which the systemic arterial pressure and the temporal progression of such pressure are determined indirectly. A valve-controlled bypass system which goes around a blood pumping unit is provided so that the blood flow in the treatment device is not interrupted and alarms are suppressed.

DocumentUS20130110028A1 discloses a valve arrangement for use in an extracorporeal blood circuit. The valve arrangement has a first valve arranged in the arterial blood line, a second valve arranged in the venous blood line, a fourth valve arranged in a first arteriovenous connection line between the arterial blood line of the circuit and the venous blood line of the circuit and a fifth valve arranged in a second arteriovenous connection line between the arterial blood line and the venous blood line and/or a third valve arranged for establishing a fluid connection in a blood line between the arterial blood line and the venous blood line of the circuit and/or a sixth valve arranged between a blood treatment device and an air venting device of the circuit.

Document FR 2889451A1 discloses an extracorporeal fluid circulating device for realizing hemodialysis operation. The device has two tubes each having ends connected to one of two channels of a catheter and ends connected to one of two channels of a dialysis machine. Junctions are placed between the ends of the tubes, where two of the junctions are connected to the other two junctions by additional tubes. A closing unit closes simultaneously the tubes or two portions of the tubes each situated between the junctions. It is a further object providing an add-on module allowing to control its configurations in automated manner.

It is also an object of the present invention to provide an add-on module enabling offering the add-on features as a supplemental option with respect to the apparatus.

It is a further object providing an add-on module allowing a single person to handle the patient's vascular access in safe manner and in aseptic conditions.

It is a further object providing an add-on module offering the possibility to have some commands located conveniently nearby the patient access.

It is a further object providing an add-on module offering the possibility to operate disinfection procedures in aseptic conditions.

It is a further object providing an add-on module which is structurally simple, cost effective and reliable.

It is a further object providing an add-on module able to prevent clotting and/or to avoid high pressure losses and/or requiring minimal amounts of fluid needed for flushing said add-on module.

### SUMMARY

At least one of the above objects is substantially reached by an add-on module for an apparatus for extracorporeal treatment of blood and by a blood set according to one or more of the appended claims.

An add-on module for an apparatus for extracorporeal treatment of blood, an apparatus (not claimed) for extracorporeal treatment of blood and a method (not claimed) for diverting a flow of liquid and/or blood in an apparatus for extracorporeal treatment of blood capable of achieving one or more of the above objects are here below described.

A 1^{st} aspect concerns an add-on module for an apparatus for extracorporeal treatment of blood

### according to claim 1.

A 2^{nd} aspect concerns a blood set for an apparatus for extracorporeal treatment of blood according to claim 15.

In a further aspect (not claimed), an apparatus for extracorporeal treatment of blood is provided comprising the blood set of the previous aspect.

A 3^{rd} aspect (not claimed) concerns a method for diverting a flow of liquid and/or blood in an apparatus for extracorporeal treatment of blood without disconnecting the patient, comprising: placing in-line an add-on module between the apparatus for extracorporeal treatment of blood and a vascular access of a patient, wherein the add-on module is according to the preceding 1^{st} aspect

### DESCRIPTION OF THE DRAWINGS

Aspects of the invention are shown in the attached drawings, which are provided by way of non-limiting example, wherein:
extracorporeal treatment of blood comprising an add-on module according to the invention;
Figure 2 shows an enlarged view of the add-on module of Figure 1;
Figure 3 shows another embodiment of the add-on module of the invention;
Figure 4 shows a variant of the add-on module of Figure 2;
Figures 5 to 11 show respective working configurations of the add-on module of Figure 1;
Figures 12 to 15 show flowcharts of respective operating modes of the of the add-on module of the invention.

### DETAILED DESCRIPTION

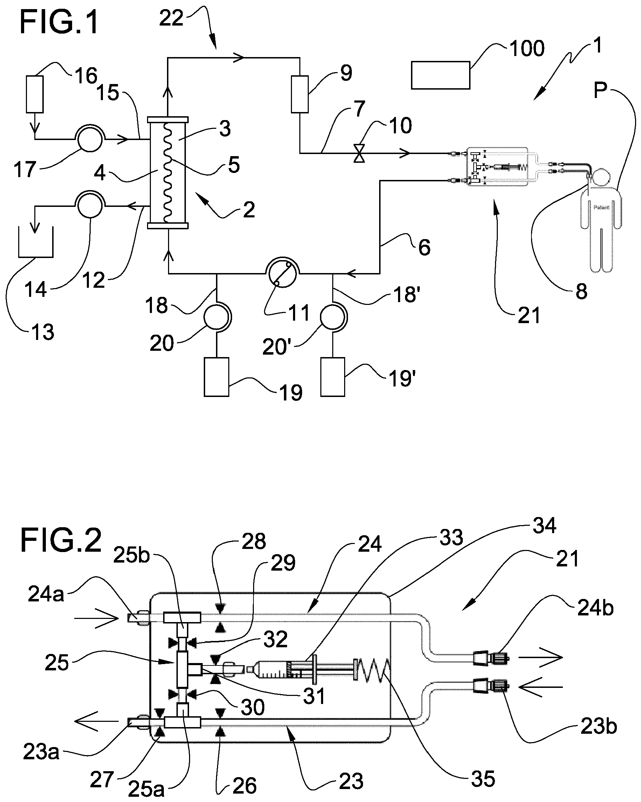

Non-limiting embodiments of an add-on module - which may implement innovative aspects of the invention - for an apparatus 1 for extracorporeal treatment of blood are shown in Figures 2, 3 and 4. In below description and in Figures 2, 3 and 4 same components are identified by same reference numerals.

An apparatus for the extracorporeal treatment of blood 1 is represented in Figure 1. The apparatus 1 of Figure 1 comprises a blood treatment unit 2 (such as a hemofilter, an ultrafilter, an hemodiafilter, a dialyzer, a plasmafilter and the like) having a primary chamber 3 and a secondary chamber 4 separated by a semipermeable membrane 5. Depending upon the treatment, the semipermeable membrane 5 of the blood treatment unit 2 may be selected to have different properties and performances.

A blood withdrawal line 6 is connected to an inlet of the primary chamber 3 and a blood return line 7 is connected to an outlet of the primary chamber 3. In use, the blood withdrawal line 6 and the blood return line 7 are connected to a needle or to a catheter or other access device 8 which is then placed in fluid communication with the patient "P" vascular system, such that blood may be withdrawn through the blood withdrawal line 6, flown through the primary chamber 3 and then returned to the patient's vascular system through the blood return line 7. An air separator, such as a deaeration chamber 9, may be present on the blood return line 7. Moreover, a monitor valve 10 may be present on the blood return line 7, downstream the deaeration chamber 9.

The blood flow through the blood lines is controlled by a blood pump 11, for instance a peristaltic blood pump, acting either on the blood withdrawal line 6 or on the blood return line 7. The embodiment of Figure 1 shows the blood pump 11 coupled to a pump section of the withdrawal line 6. A control unit 100 is connected and controls the blood pump 11 to regulate a blood flow rate.

An effluent fluid line 12 or spent dialysate line may be connected, at one end, to a fluid outlet of the secondary chamber 4 and, at its other end, to a waste which may be a discharge conduit or an effluent fluid container 13 collecting the effluent fluid extracted from the secondary chamber 4. An effluent pump 14 that operates on the effluent fluid line 13 under the control of the control unit 100 to regulate a flow rate of the effluent fluid through the effluent fluid line.

The apparatus of Figure 1 includes a dialysis fluid line 15 connected at one end with a fresh dialysis liquid source 16 and at its other end with a fluid inlet of the secondary chamber 4 of the treatment unit 2 for supplying fresh dialysis liquid to the secondary chamber 4. A dialysis fluid pump 17 is operative on the dialysis fluid line 15 under the control of the control unit 100 to supply fluid from the dialysis liquid source 16 to the secondary chamber 4 and to regulate the flow rate of the dialysis liquid.

The embodiment of Figure 1 further presents an infusion line 18 connected to the blood withdrawal line 6 between the blood pump 11 and the treatment unit 2. This infusion line 18 supplies replacement fluid from an infusion fluid container 19 connected at one end of the infusion line 18. Note that, alternatively or in addition to the infusion line 18, the apparatus of Figure 1 may include a post-dilution fluid line (not shown) connecting an infusion fluid container to the blood return line 7 and/or a pre-blood pump infusion line 18' with its own pre-blood pump infusion fluid container 19'. Furthermore, an infusion pump 20 operates on the infusion line 18 and an infusion pump 20' operates on the pre-blood pump infusion line 18' to regulate respective flow rates through the infusion lines 18, 18'. Also the infusion pumps 20, 20' are operative under the control of the control unit 100.

The effluent fluid line 12, the dialysis fluid line 15 and the secondary chamber 4 of the blood treatment unit 2 are part of a fluid circuit of the apparatus 1. The blood withdrawal line 6, the blood return line 7, the primary chamber 3 of the treatment unit 2 form part of an extracorporeal blood circuit of the apparatus 1. The infusion lines 18, 18' form part of an infusion circuit of the apparatus 1.

The control unit 100 may comprise a digital processor (CPU) with memory (or memories), an analogical type circuit, or a combination of one or more digital processing units with one or more analogical processing circuits. In the present description and in the claims it is indicated that the control unit 100 is "configured" or "programmed" to execute steps: this may be achieved in practice by any means which allow configuring or programming the control unit 100. For instance, in case of a control unit 100 comprising one or more CPUs, one or more programs are stored in an appropriate memory: the program or programs containing instructions which, when executed by the control unit 100, cause the control unit 100 to execute the steps described and/or claimed in connection with the control unit 100. Alternatively, if the control unit 100 is of an analogical type, then the circuitry of the control unit 100 is designed to include circuitry configured, in use, to process electric signals such as to execute the control unit 100 steps herein disclosed.

The control unit 100 may also be operatively connected to sensors (like flow sensors and/or pressure sensors) on the blood circuit and/or fluid circuit and/or infusion circuit. The control unit 100 is also operatively connected to clamps and valves, like the monitor valve 10. The control unit 100 may also be connected to a user interface, not shown, for instance a graphic user interface, which receives operator's inputs and displays the apparatus outputs. For instance, the graphic user interface may include a touch screen, a display screen and hard keys for entering user's inputs or a combination thereof. During extracorporeal blood treatment, the control unit 100 is configured to control at least the pumps 11, 14, 17, 20, 20' to make sure that a prefixed patient net fluid removal is achieved in the course of a treatment time, as required by a prescription provided to the control unit 100, e.g. via the user interface.

The apparatus 1 comprises a main portion 22 comprising at least a casing for the control unit 100, the control unit 100, the user interface, the blood pump 11, the effluent pump 14, the dialysis fluid pump 17, the infusion pumps 20, 20' and devices to mount a disposable set (comprising the blood treatment unit 2, the extracorporeal blood circuit, the infusion circuit and the fluid circuit) to the main portion 22.

The apparatus for the extracorporeal treatment of blood 1 shown in Figure 1 further comprises an add-on module 21 which is configured to be placed in-line between the main portion 22 of the apparatus 1 and the vascular access of a patient "P", in particular the access device 8 (e.g. a needle or a catheter) placed in fluid communication with the patient "P" vascular system. The add-on module 21 may be placed in-line on the withdrawal line 6 and on the return line 7 and closer to the access device 8 than to the main portion 22 of the apparatus 1 for extracorporeal treatment of blood. The add-on module 21 may be disposable, at least in part. The add-on module 21 comprises a substantially H-shaped conduits assembly comprising a withdrawal conduit 23, a return conduit 24 and a bridging conduit 25 connecting the withdrawal conduit 23 to the return conduit 24. Each of the withdrawal conduit 23, the return conduit 24 and the bridging conduit 25 of the embodiments shown in the annexed Figures is a straight plastic tube.The withdrawal conduit 23 is parallel to the return conduit 24 while the bridging conduit 25 is perpendicular to the withdrawal conduit 23 and to the return conduit 24.

The withdrawal conduit 23 has a first extremity 23a and an opposite second extremity 23b each provided with a quick connector, such as a Luer connector, to allow easy and quick connection/disconnection to/from the remaining portions of the withdrawal line 6. The first extremity 23a is connected or configured to be connected to a portion of the withdrawal line 6 comprising the pump section coupled to the blood pump 11. The second extremity 23b is connected or configured to be connected to a portion of the withdrawal line connected to the access device 8. The return conduit 24 has a first extremity 24a and an opposite second extremity 24b each provided with a quick connector, such as a Luer connector, to allow easy and quick connection/disconnection to/from the remaining portions of the return line 7. The first extremity 24a is connected or configured to be connected to a portion of the return line 7 comprising the deaeration chamber 9 and the monitor valve 10. The second extremity 24b is connected or configured to be connected to a portion of the return line 7 connected to the access device 8.

The bridging conduit 25 has a first extremity 25a forming a junction to the withdrawal conduit 23 and an opposite second extremity 25b forming a junction to the return conduit 24. For instance, the bridging conduit 25, the withdrawal conduit 23 and the return conduit 24 are connected through T-connectors. The withdrawal conduit 23 comprises two branches connected to the T-connector. A branch comprising the first extremity 23a and a branch comprising the second extremity 23b. The return conduit 24 comprises two branches connected to the T-connector. A branch comprising the first extremity 24a and a branch comprising the second extremity 24b.

A plurality of valves (e.g. clamps) operate on the withdrawal conduit 23, on the return conduit 24 and on the bridging conduit 25 and are configured to divert a flow of liquid and/or blood without disconnecting the patient "P".

The add-on module 21 of the embodiments of Figures 1, 2 and 4 to 11 comprises a first withdrawal valve 26 and a second withdrawal valve 27 operating on the withdrawal conduit 23. The first withdrawal valve 26 is placed upstream, with respect to a flow of blood during treatment (flowing from the second extremity 23b to the first extremity 23a of the withdrawal conduit 23), the junction of the bridging conduit 25 to said withdrawal conduit 23 and the second withdrawal valve 27 is placed downstream, with respect to said flow of blood during treatment, the junction of the bridging conduit 25 to said withdrawal conduit 23. As disclosed in Figure 2, the first withdrawal valve 26 and the second withdrawal valve 27 are placed on opposite sides with respect to the T-connector joining the bridging conduit 25 to the withdrawal conduit 23.

The add-on module 21 of the embodiments of Figures 1, 2 and 4 to 11 comprises a single return valve 28 operating on the return conduit 24. The return valve 28 is placed downstream the junction of the bridging conduit 25 to the return conduit 24 with respect to a flow of blood during treatment (flowing from the first extremity 24a to the second extremity 24b of the return conduit 24). As disclosed in Figure 2, the return valve 28 is placed at a side of the T-connector joining the bridging conduit 25 to the return conduit 24 and on the branch comprising the second extremity 24b of said return conduit 24.

In a variant embodiment, not shown, a first return valve and a second return valve operate on the return conduit 24 and are placed on opposite sides with respect to the T-connector joining the bridging conduit 25 to the return conduit 24, similar to the first withdrawal valve 26 and the second withdrawal valve 27. The return valve placed upstream the junction of the bridging conduit 25 to the return conduit 24 with respect to the flow of blood during treatment may perform the function of the monitor valve 10. The add-on module 21 of the embodiments of Figures 1, 2 and 4 to 11 comprises a first bridging valve 29 and a second bridging valve 30 operating on the bridging conduit 25. The first bridging valve 29 is placed closer to the return conduit 24 (closer than the second bridging valve 30) and the second bridging valve 30 is placed closer to the withdrawal conduit 23 (closer than the first bridging valve 29).

The bridging conduit 25 comprises a first branch having the first extremity 25a and a second branch having the second extremity 25b. The first branch and the second branch are connected to each other through a T-connector delimiting also an access point 31 suitable to connect a liquid source. The first bridging valve 29 is placed on the second branch bridging conduit 25 and the second bridging valve 30 is placed on the first branch of the bridging conduit 25. The access point 31 is located between the first bridging valve 29 and the second bridging valve 30.

A stretch of tubing departs form the access point 31 and is provided with an access valve 32. The stretch of tubing may be connected to a liquid source 33, like a syringe, e.g. of 50 ml, or a bag, e.g. of 500 ml. The liquid of said liquid source may be saline.

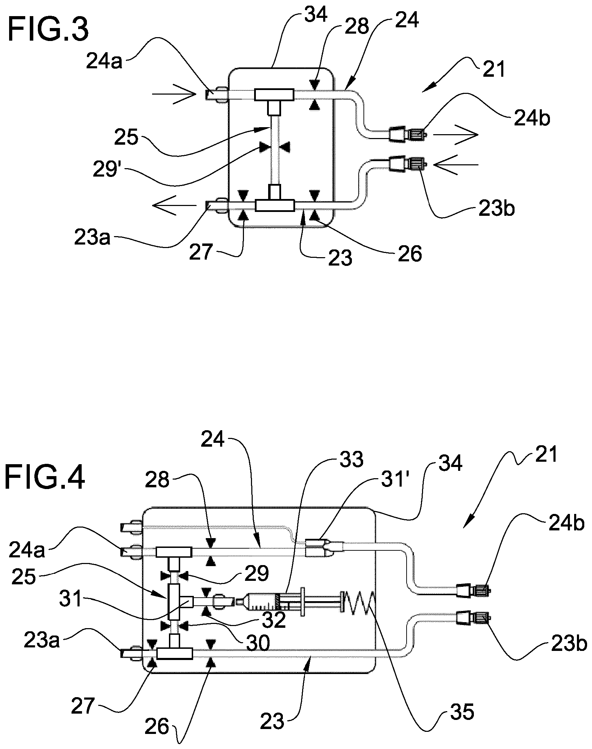

In the variant embodiment of Figure 3, the add-on module 21 comprises a single bridging valve 29' operating on the bridging conduit 25 and does not comprise any access point 31. In the variant embodiment of Figure 4, the add-on module 21 further comprises (in addition with respect to the embodiment of Figure 3) a further access point 31' on the return conduit 24, e.g. for calcium infusion. The further access point 31' is placed on the branch comprising the second extremity 24b and may be connected to a bag of the apparatus.

The substantially H-shaped conduits assembly and the plurality of valves 26, 27, 28, 29, 30, 31 are mounted on a holder 34 which may be shaped like a plate provided with seats, or other holding devices, configured to accommodate the substantially H-shaped conduits assembly and the plurality of valves 26, 27, 28, 29, 30, 31. In the embodiments of Figures 1, 2 and 4 to 11, the holder 34 comprises a seat, or other holding device to hold the syringe 33, and a spring 35 for pressurizing the syringe 33 and ejecting the fluid contained therein through the access point 31.

The add-on module 21 may also comprise one or more sensors (e.g. pressure sensor, flowmeter, air detector, etc.), not shown, active on the H-shaped conduits assembly, i.e. on the return conduit 24 and/or on the withdrawal conduit 23, on the bridging conduit 25. The plurality of valves 26, 27, 28, 29, 30, 31 and the sensor/s are operatively connected or connectable to the control unit 100 of the apparatus 1 and/or to a control unit of the add-on module 21 which may be slaved to the control unit 100 of the apparatus 1. Said control unit/s is/are configured for commanding, optionally automatically, the configurations of the plurality of valves 26, 27, 28, 29, 30, 31. The valves 26, 27, 28, 29, 30, 31 and/or the control unit of the add-on module 21 may be connected to the control unit 100 of the apparatus 1 through a wired or wireless communication device.

The add-on module 21 comprises a power supply to power the valves 26, 27, 28, 29, 30, 31 and/or the sensor/s and/or control unit or said valves 26, 27, 28, 29, 30, 31 and/or the control unit of the add-on module 21 may be connected to a power supply of the apparatus 1 to be powered by said apparatus 1.

The add-on module 21 may be a portable module and/or comprisesan attachment device configured to install said add-on module on another element, optionally in a removable manner, e.g. to clothing of a patient "P" or to a bed for the patient "P" or to a main portion of the apparatus for extracorporeal treatment of blood. The lengths of the portion of the return line 7 comprising the deaeration chamber 9 and the monitor valve 10 and of the portion of the withdrawal line 6 comprising the pump section coupled to the blood pump 11 are such to place the add-on module close to the patient "P".

In use and according to a method of the invention for diverting a flow of liquid and/or blood in the apparatus 1, before treatment of the patient "P", the blood circuit is primed and the control unit 100 controls the valves 26, 27, 28, 29, 30, 31 to allow priming the withdrawal conduit 23, the return conduit 24 and the bridging conduit 25, using either solution bags of the apparatus 1 or saline from the liquid source 33 connected to the access point 31.

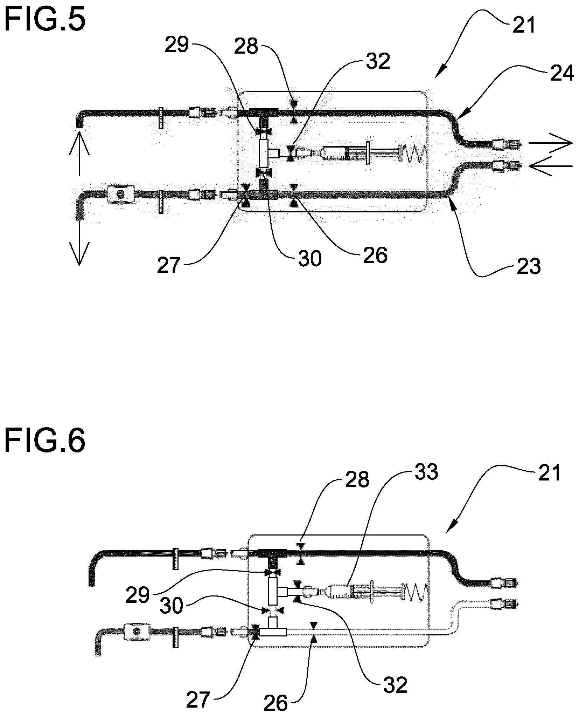

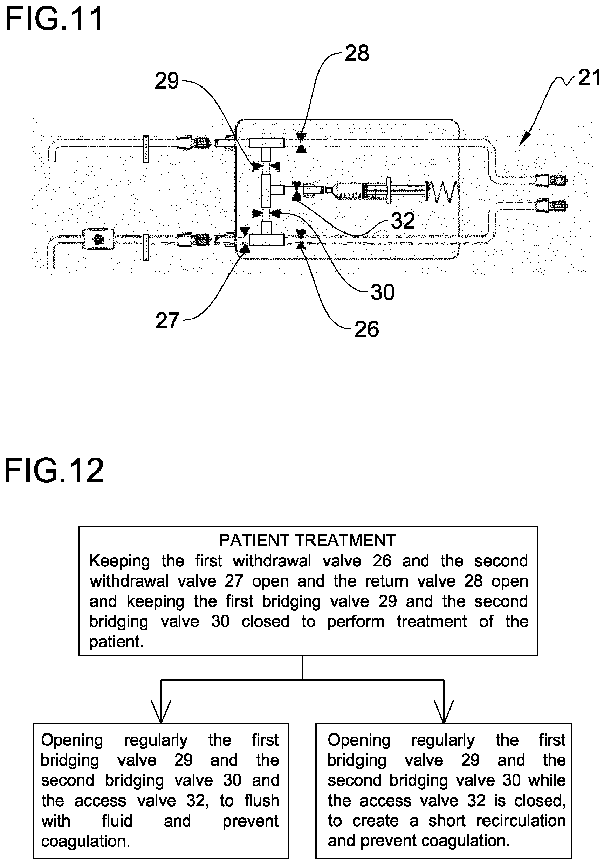

During patient treatment (Figures 5 and 12), when blood flows from patient "P" through the withdrawal line 6, into the primary chamber 3 of the blood treatment unit 2 and then back into the patient "P" through the return line 7, the control unit 100 controls the valves 26, 27, 28, 29, 30, 31 to configure said valves 26, 27, 28, 29, 30, 31 in a treatment configuration. In the treatment configuration, the first bridging valve 29, the second bridging valve 30 and the access valve 32 are closed, the first withdrawal valve 26, the second withdrawal valve 27 and the return valve 28 are open.

During patient treatment, since blood may stagnate at the closed first bridging valve 29 and the second bridging valve 30, in order to prevent coagulation, the first bridging valve 29 and the second bridging valve 30 and the access valve 32 are opened regularly (intermittently), to flush the bridging conduit 25 with fluid and prevent coagulation. According to a variant of the method, the first bridging valve 29 and the second bridging valve 30 are opened regularly (intermittently) while the access valve 31 is closed, to flush the bridging conduit 25 with blood, creating a short recirculation and preventing coagulation (Figure 12).

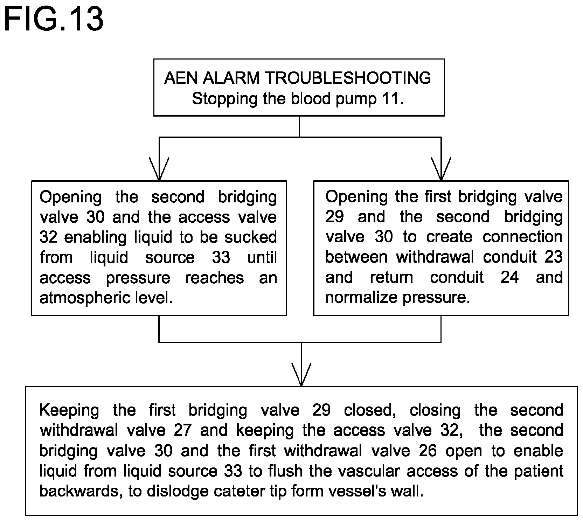

In case of Access Extremely Negative (AEN) alarm, the blood pump 11 is stopped, the second bridging valve 30 and the access valve 32 are opened to enable saline to be sucked from the syringe 33 until access pressure reaches an approximately atmospheric level or the first bridging valve 29 and the second bridging valve 30 are both opened to create connection between the withdrawal conduit 23 and the return conduit 24 and to normalize pressure. After the access pressure got closer to the atmospheric level, the first bridging valve 29 is kept closed, the second withdrawal valve 27 is closed while the access valve 32, the second bridging valve 30 and the first withdrawal valve 26 are kept open. This way, the fluid from syringe 33 flushes the vascular access of the patient "P" backwards to help dislodge the catheter tip from vessel's wall (Figures 6 and 13).

According to another possible action to solve an AEN alarm, the first withdrawal valve 26 and the second withdrawal valve 27 are closed while the other valves are open. The blood pump 11 is controlled to turn in order to increase the access negative pressure till a defined value and then stopped. Then the first withdrawal valve 26 and the second withdrawal valve 27 are opened to create a sudden negative pressure peak in the catheter's access way to move or break a clot.

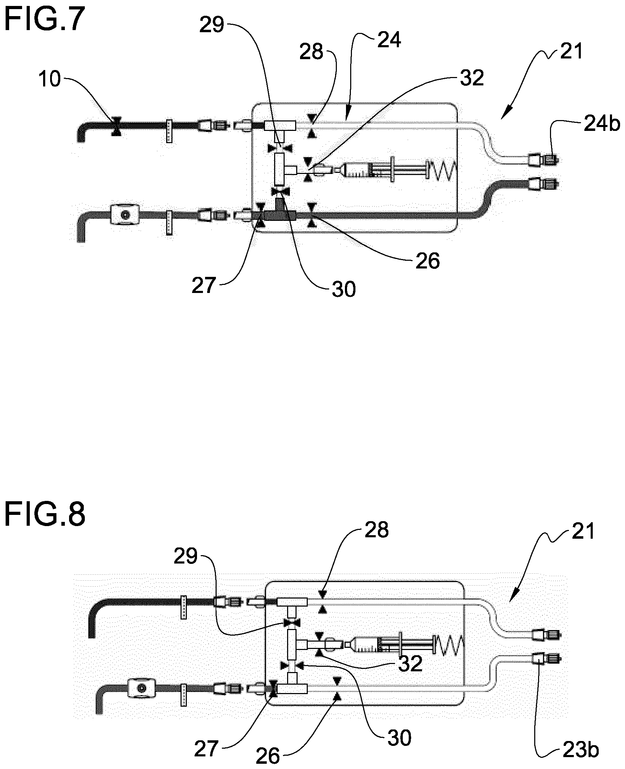

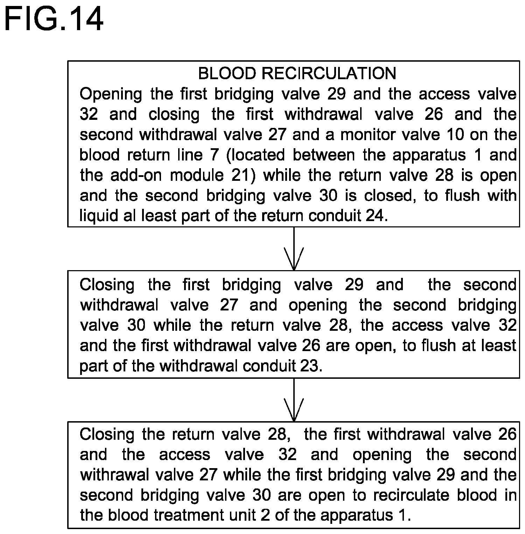

If these attempts fail, the control unit 100 controls the plurality of valves to 26, 27, 28, 29, 30, 31 to engage blood recirculation, while notifying the staff that action is required (moving the patient "P", caring for the vascular access, etc.). Prior to enabling blood recirculation, flushing is needed in the return conduit 24 and withdrawal conduit 23 to avoid clotting and to enable patient reconnection after staff intervention. To this aim, the branch of the return conduit 24 comprising the second extremity 24b is flushed with fluid by closing the second bridging valve 30 and the monitor clamp 10 while the first bridging valve 29, the first withdrawal valve 26, the second withdrawal valve 27, the return valve 28 and the access valve 32 are open and fluid from the syringe 33 is injected (Figures 7 and 14).

The branch of the withdrawal conduit 6 having the second extremity 23b is then flushed. The first bridging valve 29 and the second withdrawal valve 27 are closed, the second bridging valve 30, the first withdrawal valve 26 and the access valve 32 are open and saline from the syringe 33 is injected (Figures 8 and 14).

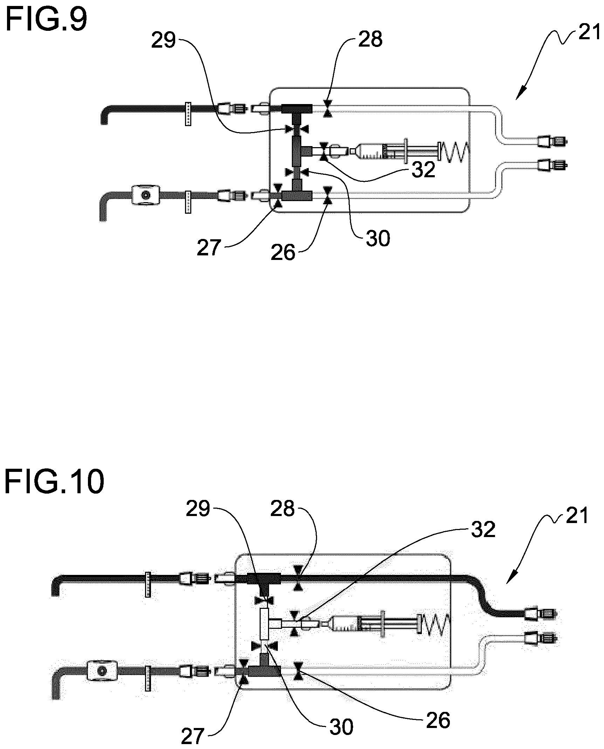

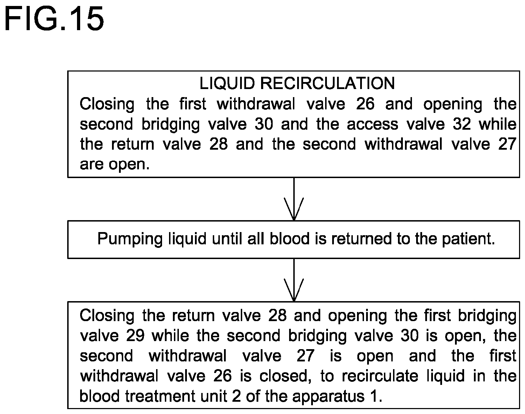

The blood recirculation starts with the return valve 28 closed, the first withdrawal valve 26 closed, the second withdrawal valve 27 open and the first and second bridging valves 29, 30 open, so that blood recirculates in the blood treatment unit 2 of the apparatus 1 and staff intervention is executed (Figures 9 and 14). At the end of the treatment, with blood return to the patient "P", saline recirculation starts. With the first withdrawal valve 26 and the first bridging valve 29 closed and the second bridging valve 30, the return valve 28, the second withdrawal valve 27 and the access valve 32 open, saline is pumped by the blood pump 11 until all blood is returned to the patient "P". Then, the return valve 28 is closed and the first bridging valve 29 is opened while the second bridging valve 30 is still open, the second withdrawal valve 27 is open and the first withdrawal valve 26 is closed, to recirculate liquid in the blood treatment unit 2 of the apparatus 1 (Figures 11 and 15).

While the invention has been described in connection with what is presently considered to be the most practical and preferred embodiments, it is to be understood that the invention is not to be limited to the disclosed embodiments, but on the contrary, is intended to cover various modifications and equivalent arrangements included within the scope of the appended claims.

## Claims

1. Add-on module for an apparatus for extracorporeal treatment of blood, wherein the apparatus (1) comprises:
a blood treatment unit (2);
an extracorporeal blood circuit coupled to the blood treatment unit (2) and comprising a blood withdrawal line (6) and a blood return line (7) connectable to a vascular access of a patient (P);
a blood pump (11) configured to be coupled to a pump section of the extracorporeal blood circuit;
wherein the add-on module (21) is configured to be placed in-line between a main portion (22) of the apparatus (1) and the vascular access of the patient (P) and comprises:
- a substantially H-shaped conduits assembly comprising a withdrawal conduit (23), a return conduit (24) and a bridging conduit (25) connecting the withdrawal conduit (23) to the return conduit (24); wherein the withdrawal conduit (23) is connectable upstream and downstream to the withdrawal line (6), wherein the return conduit (24) is connectable upstream and downstream to the return line (7);
- a plurality of valves (26, 27, 28, 29, 30, 31) operating on the withdrawal conduit (23), on the return conduit (24) and on the bridging conduit (25) and configured to divert a flow of liquid and/or blood without disconnecting the patient (P);
wherein the plurality of valves (26, 27, 28, 29, 30, 31) comprises at least a withdrawal valve (26, 27) operating on the withdrawal conduit (23), at least a return valve (28) operating on the return conduit (24) and at least a bridging valve (29, 30) operating on the bridging conduit (25);
**characterized in that** said at least a withdrawal valve (26, 27) comprises a first withdrawal valve (26) and a second withdrawal valve (27) operating on the withdrawal conduit (23), wherein the first withdrawal valve (26) and the second withdrawal valve (27) are placed on opposite sides with respect to a junction of the bridging conduit (25) to the withdrawal conduit (23), wherein the first withdrawal valve (26) is placed upstream, with respect to a flow of blood during treatment, the junction of the bridging conduit (25) to said withdrawal conduit (23), wherein the second withdrawal valve (27) is placed downstream, with respect to the flow of blood during treatment, the junction of the bridging conduit (25) to said withdrawal conduit (23).

2. The add-on module according to claim 1, wherein the withdrawal conduit (23) is substantially parallel to the return conduit (24) and wherein the bridging conduit (25) is transversal, optionally perpendicular, to the withdrawal conduit (23) and to the return conduit (24).

3. The add-on module according to claim 1 or 2, wherein at least one of the withdrawal conduit (23), the return conduit (24) and the bridging conduit (25) is straight.

4. The add-on module according to any of claims 1 to 3, wherein the valves (26, 27, 28, 29, 30, 31) are clamps.

5. The add-on module according to any of claims 1 to 4, wherein the return valve (28) is placed downstream a junction of the bridging conduit (25) to the return conduit (24) with respect to a flow of blood during treatment.

6. The add-on module according to any of claims 1 to 5, wherein said at least a bridging valve (29, 30) comprises a first bridging valve (29) and a second bridging valve (30) operating on the bridging conduit (25), wherein the first bridging valve (29) is placed closer to the return conduit (24) and the second bridging valve (30) is placed closer to the withdrawal conduit (23), wherein the bridging conduit (25) has an access point (31) located between the first bridging valve (29) and the second bridging valve (30) and suitable to connect a liquid source (33), optionally a saline source, optionally a syringe or a bag, or optionally a citrate source coming from the apparatus (1) for extracorporeal blood treatment.

7. The add-on module according to claim 6, comprising a liquid source, optionally a syringe or a bag, connected or connectable to the access point (31).

8. The add-on module according to any of claims 1 to 7, comprising a holder (34) configured to hold the substantially H-shaped conduits assembly and the plurality of valves (26, 27, 28, 29, 30, 31).

9. The add-on module according to claim 8 when depending on claim 7, wherein the holder (34) is configured to hold the liquid source (33) .

10. The add-on module according to any of claims 1 to 9, wherein the plurality of valves (26, 27, 28, 29, 30, 31) is configured to be arranged at least in a treatment configuration, in which said at least a withdrawal valve (26, 27) and said at least a return valve (28) are open and said at least a bridging valve (29, 30) is closed, to perform treatment of the patient (P).

11. The add-on module according to claim 5, wherein the plurality of valves (26, 27, 28, 29, 30, 31) is configured to be arranged at least in a recirculation configuration, in which the return valve (28) is closed, the first withdrawal valve (26) is closed, the second withdrawal valve (27) is open and said at least a bridging valve (29, 30) is open, so that blood or liquid recirculates in the blood treatment unit (2) of the apparatus (1) without disconnecting the patient (P).

12. The add-on module according to claim 6, wherein the plurality of valves (26, 27, 28, 29, 30, 31) is configured to be arranged in a return branch flushing configuration, in which the first bridging valve (29) is open, the second bridging valve (30) is closed and the liquid source (33) is connected to the access point (31) to inject liquid and flush a branch of the return conduit (24) placed downstream a junction of the bridging conduit (25) to the return conduit (24).

13. The add-on module according to claim 12, wherein the plurality of valves (26, 27, 28, 29, 30, 31) is configured to be arranged in a withdrawal branch flushing configuration, in which the first bridging valve (29) is closed, the second bridging valve (30) is open, the second withdrawal valve (27) is closed and the liquid source (33) is connected to the access point (31) to inject liquid and flush a branch of the withdrawal conduit (23) placed upstream a junction of the bridging conduit (25) to the withdrawal conduit (23) .

14. The add-on module according to any of claims 10 to 13, wherein the valves of the plurality of valves (26, 27, 28, 29, 30, 31) are operatively connected or connectable to a control unit (100), optionally of the apparatus (1) for extracorporeal treatment of blood, or wherein the add-on module (21) comprises a control unit operatively connected to the valves of the plurality of valves (26, 27, 28, 29, 30, 31); wherein the control unit (100) is configured for commanding, optionally automatically, the configurations of the plurality of valves (26, 27, 28, 29, 30, 31) .

15. A blood set for an apparatus for extracorporeal treatment of blood, the blood set comprising:
a blood treatment unit (2);
an extracorporeal blood circuit coupled to the blood treatment unit (2) and comprising a blood withdrawal line (6) and a blood return line (7) connectable to a vascular access of a patient (P), optionally each of the blood withdrawal line (6) and the blood return line (7) having a respective end connector configured to be connected to the vascular access of the patient (P);
an add-on module (21) according to one of the preceding claims 1 to 14, wherein the add-on module (21) is placed or is configured to be placed in-line between a main portion (22) of the apparatus (1) for extracorporeal treatment of blood and the vascular access of the patient (P), in particular the withdrawal conduit (23) and the return conduit (24) having a respective end counter-connector configured to be coupled or coupled with the end connector of the blood withdrawal line (6) and of the blood return line (7).

## Patentansprüche

1. Zusatzmodul für eine Vorrichtung zur extrakorporale Blutbehandlung, wobei die Vorrichtung (1) Folgendes umfasst:
eine Blutbehandlungseinheit (2),
einen extrakorporalen Blutkreislauf, der an die Blutbehandlungseinheit (2) gekoppelt ist und eine Blutentnahmeleitung (6) und eine Blutrückgabeleitung (7) umfasst, die mit einem Gefäßzugang eines Patienten (P) verbindbar sind,
eine Blutpumpe (11), die zum Koppeln an einen Pumpenabschnitt des extrakorporalen Blutkreislaufs ausgestaltet ist,
wobei das Zusatzmodul (21) dazu ausgestaltet ist, in-line zwischen einen Hauptabschnitt (22) der Vorrichtung (1) und den Gefäßzugang des Patienten (P) platziert zu werden, und Folgendes umfasst:
- eine im Wesentlichen H-förmige Kanalbaugruppe, die einen Entnahmekanal (23), einen Rückgabekanal (24) und einen Überbrückungskanal (25) umfasst, der den Entnahmekanal (23) mit dem Rückgabekanal (24) verbindet, wobei der Entnahmekanal (23) stromaufwärts und stromabwärts mit der Entnahmeleitung (6) verbindbar ist, wobei der Rückgabekanal (24) stromaufwärts und stromabwärts mit der Rückgabeleitung (7) verbindbar ist,
- eine Vielzahl von Ventilen (26, 27, 28, 29, 30, 31), die an dem dem Entnahmekanal (23), an dem Rückgabekanal (24) und an dem Überbrückungskanal (25) wirken und dazu ausgestaltet sind, einen Flüssigkeits- und/oder Blutstrom ohne Abtrennung von dem Patienten (P) umzuleiten,
wobei die Vielzahl von Ventilen (26, 27, 28, 29, 30, 31) mindestens ein Entnahmeventil (26, 27), das an dem Entnahmekanal (23) wirkt, mindestens ein Rückgabeventil (28), das an dem Rückgabekanal (24) wirkt, und mindestens ein Überbrückungsventil (29, 30), das an dem Überbrückungskanal (25) wirkt, umfassen,
**dadurch gekennzeichnet, dass** das mindestens eine Entnahmeventil (26, 27) ein erstes Entnahmeventil (26) und ein zweites Entnahmeventil (27) umfasst, die an dem Entnahmekanal (23) wirken, wobei das erste Entnahmeventil (26) und das zweite Entnahmeventil (27) bezüglich eines Übergangs des Überbrückungskanals (25) zu dem Entnahmekanal (23) an gegenüberliegenden Seiten platziert sind, wobei das erste Entnahmeventil (26) bezüglich eines Blutstroms während der Behandlung stromaufwärts von dem Übergang des Überbrückungskanals (25) zu dem Entnahmekanal (23) platziert ist, wobei das zweite Entnahmeventil (27) bezüglich eines Blutstroms während der Behandlung stromabwärts von dem Übergang des Überbrückungskanals (25) zu dem Entnahmekanal (23) platziert ist.

2. Zusatzmodul nach Anspruch 1, wobei der Entnahmekanal (23) im Wesentlichen parallel zu dem Rückgabekanal (24) verläuft und wobei der Überbrückungskanal (25) quer, optional senkrecht, zu dem Entnahmekanal (23) und dem Rückgabekanal (24) verläuft.

3. Zusatzmodul nach Anspruch 1 oder 2, wobei der Entnahmekanal (23) und/oder der Rückgabekanal (24) und/oder der Überbrückungskanal (25) gerade sind.

4. Zusatzmodul nach einem der Ansprüche 1 bis 3, wobei die Ventile (26, 27, 28, 29, 30, 31) Klemmen sind.

5. Zusatzmodul nach einem der Ansprüche 1 bis 4, wobei das Rückgabeventil (28) bezüglich eines Blutstroms während der Behandlung stromabwärts von einem Übergang des Überbrückungskanals (25) zu dem Rückgabekanal (24) platziert ist.

6. Zusatzmodul nach einem der Ansprüche 1 bis 5, wobei das mindestens eine Überbrückungsventil (29, 30) ein erstes Überbrückungsventil (29) und ein zweites Überbrückungsventil (30) umfasst, die an dem Überbrückungskanal (25) wirken, wobei das erste Überbrückungsventil (29) näher an dem Rückgabekanal (24) platziert ist und das zweite Überbrückungsventil (30) näher an dem Entnahmekanal (23) platziert ist, wobei der Überbrückungskanal (25) einen Zugangspunkt (31) hat, der zwischen dem ersten Überbrückungsventil (29) und dem zweiten Überbrückungsventil (30) angeordnet ist und an den eine Flüssigkeitsquelle (33), optional eine Kochsalzlösungsquelle, optional eine Spritze oder ein Beutel oder optional eine Citratquelle angeschlossen werden kann, die von der Vorrichtung (1) zur extrakorporalen Blutbehandlung kommen.

7. Zusatzmodul nach Anspruch 6, umfassend eine Flüssigkeitsquelle, optional eine Spritze oder einen Beutel, der bzw. die mit dem Zugangspunkt (31) verbunden oder verbindbar ist.

8. Zusatzmodul nach einem der Ansprüche 1 bis 7, umfassend einen Halter (34), der zum Halten der im Wesentlichen H-förmigen Kanalbaugruppe und der Vielzahl von Ventilen (26, 27, 28, 29, 30, 31) ausgestaltet ist.

9. Zusatzmodul nach Anspruch 8, wenn von Anspruch 7 abhängig, wobei der Halter (34) zum Halten der Flüssigkeitsquelle (33) ausgestaltet ist.

10. Zusatzmodul nach einem der Ansprüche 1 bis 9, wobei die Vielzahl von Ventilen (26, 27, 28, 29, 30, 31) dazu ausgestaltet sind, mindestens in einer Behandlungskonfiguration angeordnet zu sein, in der das mindestens eine Entnahmeventil (26, 27) und das mindestens eine Rückgabeventil (28) offen sind und das mindestens eine Überbrückungsventil (29, 30) geschlossen ist, um die Behandlung des Patienten (P) durchzuführen.

11. Zusatzmodul nach Anspruch 5, wobei die Vielzahl von Ventilen (26, 27, 28, 29, 30, 31) dazu ausgestaltet sind, mindestens in einer Rezirkulierungskonfiguration angeordnet zu sein, in der das Rückgabeventil (28) geschlossen ist, das erste Entnahmeventil (26) geschlossen ist, das zweite Entnahmeventil (27) offen ist und das mindestens eine Überbrückungsventil (29, 30) offen ist, so dass Blut oder Flüssigkeit in der Blutbehandlungseinheit (2) der Vorrichtung (1) ohne Abtrennung von dem Patienten (P) rezirkuliert.

12. Zusatzmodul nach Anspruch 6, wobei die Vielzahl von Ventilen (26, 27, 28, 29, 30, 31) dazu ausgestaltet sind, in einer Rückgabezweigspülkonfiguration angeordnet zu sein, in der das erste Überbrückungsventil (29) offen ist, das zweite Überbrückungsventil (30) geschlossen ist und die Flüssigkeitsquelle (33) mit dem Zugangspunkt (31) verbunden ist, um Flüssigkeit einzuführen und einen Zweig des Rückgabekanals (24), der stromabwärts von einem Übergang des Überbrückungskanals (25) zu dem Rückgabekanal (24) platziert ist, zu spülen.

13. Zusatzmodul nach Anspruch 12, wobei die Vielzahl von Ventilen (26, 27, 28, 29, 30, 31) dazu ausgestaltet sind, in einer Entnahmezweigspülkonfiguration angeordnet zu sein, in der das erste Überbrückungsventil (29) geschlossen ist, das zweite Überbrückungsventil (30) offen ist, das zweite Entnahmeventil (27) geschlossen ist und die Flüssigkeitsquelle (33) mit dem Zugangspunkt (31) verbunden ist, um Flüssigkeit einzuführen und einen Zweig des Entnahmekanals (23), der stromaufwärts von einem Übergang des Überbrückungskanals (25) zu dem Entnahmekanal (23) platziert ist, zu spülen.

14. Zusatzmodul nach einem der Ansprüche 10 bis 13, wobei die Ventile der Vielzahl von Ventilen (26, 27, 28, 29, 30, 31) mit einer Steuereinheit (100), optional der Vorrichtung (1) für die extrakorporale Blutbehandlung, wirkverbunden oder wirkverbindbar sind oder wobei das Zusatzmodul (21) eine mit den Ventilen der Vielzahl von Ventilen (26, 27, 28, 29, 30, 31) wirkverbundene Steuereinheit umfasst, wobei die Steuereinheit (100) dazu ausgestaltet ist, die Konfigurationen der Vielzahl von Ventilen (26, 27, 28, 29, 30, 31) optional automatisch zu steuern.

15. Blutsatz für eine Vorrichtung für die extrakorporale Blutbehandlung, wobei der Blutsatz Folgendes umfasst:
eine Blutbehandlungseinheit (2),
einen extrakorporalen Blutkreislauf, der an die Blutbehandlungseinheit (2) gekoppelt ist und eine Blutentnahmeleitung (6) und eine Blutrückgabeleitung (7) umfasst, die mit einem Gefäßzugang eines Patienten (P) verbindbar sind, optional wobei die Blutentnahmeleitung (6) und die Blutrückgabeleitung (7) jeweils einen jeweiligen Endverbinder haben, der zur Verbindung mit dem Gefäßzugang des Patienten (P) ausgestaltet ist,
ein Zusatzmodul (21) nach einem der vorhergehenden Ansprüche 1 bis 14, wobei das Zusatzmodul (21) in-line zwischen einen Hauptabschnitt (22) der Vorrichtung (1) für die extrakorporale Blutbehandlung und den Gefäßzugang des Patienten (P) platziert ist oder dazu ausgestaltet ist, dort platziert zu werden, wobei insbesondere der Entnahmekanal (23) und der Rückgabekanal (24) einen jeweiligen Endgegenverbinder haben, der zum Koppeln mit dem Endverbinder der Blutentnahmeleitung (6) und der Blutrückgabeleitung (7) ausgestaltet ist oder mit diesem gekoppelt ist.

## Revendications

1. Module complémentaire pour un appareil de traitement extracorporel du sang, l'appareil (1) comprenant :
une unité de traitement du sang (2) ;
un circuit sanguin extracorporel accouplé à l'unité de traitement du sang (2) et comprenant une ligne de prélèvement de sang (6) et une ligne de retour de sang (7) pouvant être raccordées à un accès vasculaire d'un patient (P) ;
une pompe à sang (11) conçue pour être accouplée à une section de pompe du circuit sanguin extracorporel ;
le module complémentaire (21) étant conçu pour être placé en ligne entre une partie principale (22) de l'appareil (1) et l'accès vasculaire du patient (P) et comprenant :
un ensemble de conduits sensiblement en forme de H comprenant un conduit de prélèvement (23), un conduit de retour (24) et un conduit de liaison (25) reliant le conduit de prélèvement (23) au conduit de retour (24) ; le conduit de prélèvement (23) pouvant être raccordé en amont et en aval à la ligne de prélèvement (6), le conduit de retour (24) pouvant être raccordé en amont et en aval à la ligne de retour (7) ;
une pluralité de vannes (26, 27, 28, 29, 30, 31) fonctionnant sur le conduit de prélèvement (23), sur le conduit de retour (24) et sur le conduit de liaison (25) et conçues pour dévier un flux de liquide et/ou de sang sans débrancher le patient (P) ;
la pluralité de vannes (26, 27, 28, 29, 30, 31) comprenant au moins une vanne de prélèvement (26, 27) fonctionnant sur le conduit de prélèvement (23), au moins une vanne de retour (28) fonctionnant sur le conduit de retour (24) et au moins une vanne de liaison (29, 30) fonctionnant sur le conduit de liaison (25) ;
**caractérisé en ce que** ladite au moins une vanne de prélèvement (26, 27) comprend une première vanne de prélèvement (26) et une deuxième vanne de prélèvement (27) fonctionnant sur le conduit de prélèvement (23), la première vanne de prélèvement (26) et la deuxième vanne de prélèvement (27) étant placées sur des côtés opposés par rapport à une jonction du conduit de liaison (25) avec le conduit de prélèvement (23), la première vanne de prélèvement (26) étant placée en amont, par rapport à un flux de sang pendant le traitement, de la jonction du conduit de liaison (25) avec ledit conduit de prélèvement (23), la deuxième vanne de prélèvement (27) étant placée en aval, par rapport au flux de sang pendant le traitement, de la jonction du conduit de liaison (25) avec ledit conduit de prélèvement (23).

2. Module complémentaire selon la revendication 1, le conduit de prélèvement (23) étant sensiblement parallèle au conduit de retour (24) et le conduit de liaison (25) étant transversal, éventuellement perpendiculaire, au conduit de prélèvement (23) et au conduit de retour (24).

3. Module complémentaire selon la revendication 1 ou 2, au moins l'un parmi le conduit de prélèvement (23), le conduit de retour (24) et le conduit de liaison (25) étant droit.

4. Module complémentaire selon l'une quelconque des revendications 1 à 3, les vannes (26, 27, 28, 29, 30, 31) étant des pinces.

5. Module complémentaire selon l'une quelconque des revendications 1 à 4, la vanne de retour (28) étant placée en aval d'une jonction du conduit de liaison (25) avec le conduit de retour (24) par rapport à un flux de sang pendant le traitement.

6. Module complémentaire selon l'une quelconque des revendications 1 à 5, ladite au moins une vanne de liaison (29, 30) comprenant une première vanne de liaison (29) et une deuxième vanne de liaison (30) fonctionnant sur le conduit de liaison (25), la première vanne de liaison (29) étant placée plus près du conduit de retour (24) et la deuxième vanne de liaison (30) étant placée plus près du conduit de prélèvement (23), le conduit de liaison (25) ayant un point d'accès (31) situé entre la première vanne de liaison (29) et la deuxième vanne de liaison (30) et approprié pour raccorder une source de liquide (33), éventuellement une source de solution saline, éventuellement une seringue ou une poche, ou éventuellement une source de citrate venant de l'appareil (1) de traitement extracorporel du sang.

7. Module complémentaire selon la revendication 6, comprenant une source de liquide, éventuellement une seringue ou une poche, raccordée ou pouvant être raccordée au point d'accès (31).

8. Module complémentaire selon l'une quelconque des revendications 1 à 7, comprenant un support (34) conçu pour supporter l'ensemble de conduits sensiblement en forme de H et la pluralité de vannes (26, 27, 28, 29, 30, 31) .

9. Module complémentaire selon la revendication 8 lorsqu'elle dépend de la revendication 7, le support (34) étant conçu pour supporter la source de liquide (33).

10. Module complémentaire selon l'une quelconque des revendications 1 à 9, la pluralité de vannes (26, 27, 28, 29, 30, 31) étant conçue pour être agencée au moins dans une configuration de traitement, dans laquelle ladite au moins une vanne de prélèvement (26, 27) et ladite au moins une vanne de retour (28) sont ouvertes et ladite au moins une vanne de liaison (29, 30) est fermée, pour réaliser le traitement du patient (P).

11. Module complémentaire selon la revendication 5, la pluralité de vannes (26, 27, 28, 29, 30, 31) étant conçue pour être agencée au moins dans une configuration de recirculation, dans laquelle la vanne de retour (28) est fermée, la première vanne de prélèvement (26) est fermée, la deuxième vanne de prélèvement (27) est ouverte et ladite au moins une vanne de liaison (29, 30) est ouverte, de sorte que le sang ou le liquide recircule dans l'unité de traitement du sang (2) de l'appareil (1) sans débrancher le patient (P).

12. Module complémentaire selon la revendication 6, la pluralité de vannes (26, 27, 28, 29, 30, 31) étant conçue pour être agencée dans une configuration de rinçage de branche de retour, dans laquelle la première vanne de liaison (29) est ouverte, la deuxième vanne de liaison (30) est fermée et la source de liquide (33) est raccordée au point d'accès (31) pour injecter un liquide et rincer une branche du conduit de retour (24) placée en aval d'une jonction du conduit de liaison (25) avec le conduit de retour (24).

13. Module complémentaire selon la revendication 12, la pluralité de vannes (26, 27, 28, 29, 30, 31) étant conçue pour être agencée dans une configuration de rinçage de branche de prélèvement, dans laquelle la première vanne de liaison (29) est fermée, la deuxième vanne de liaison (30) est ouverte, la deuxième vanne de prélèvement (27) est fermée et la source de liquide (33) est raccordée au point d'accès (31) pour injecter un liquide et rincer une branche du conduit de prélèvement (23) placée en amont d'une jonction du conduit de liaison (25) avec le conduit de prélèvement (23).

14. Module complémentaire selon l'une quelconque des revendications 10 à 13, les vannes de la pluralité de vannes (26, 27, 28, 29, 30, 31) étant raccordées ou pouvant être raccordées de manière opérationnelle à une unité de commande (100), éventuellement de l'appareil (1) de traitement extracorporel du sang, ou le module complémentaire (21) comprenant une unité de commande raccordée de manière opérationnelle aux vannes de la pluralité de vannes (26, 27, 28, 29, 30, 31) ; l'unité de commande (100) étant conçue pour commander, éventuellement automatiquement, les configurations de la pluralité de vannes (26, 27, 28, 29, 30, 31).

15. Ensemble de lignes de sang pour un appareil de traitement extracorporel du sang, l'ensemble de lignes de sang comprenant :
une unité de traitement du sang (2) ;
un circuit sanguin extracorporel accouplé à l'unité de traitement du sang (2) et comprenant une ligne de prélèvement de sang (6) et une ligne de retour de sang (7) pouvant être raccordées à un accès vasculaire d'un patient (P), éventuellement chacune parmi la ligne de prélèvement de sang (6) et la ligne de retour de sang (7) ayant un élément de raccordement d'extrémité respectif conçu pour être raccordé à l'accès vasculaire du patient (P) ;
un module complémentaire (21) selon l'une des revendications précédentes 1 à 14, le module complémentaire (21) étant placé ou étant conçu pour être placé en ligne entre une partie principale (22) de l'appareil (1) de traitement extracorporel du sang et l'accès vasculaire du patient (P), en particulier le conduit de prélèvement (23) et le conduit de retour (24) ayant un contre-élément de raccordement d'extrémité respectif conçu pour être accouplé ou accouplé à l'élément de raccordement d'extrémité de la ligne de prélèvement de sang (6) et de la ligne de retour de sang (7) .
